# EUROPEAN PATENT APPLICATION

(11) **EP 4 616 780 A1**
(43) Date of publication of application: **17.09.2025**
(21) Application number: 23888465.4
(22) Date of filing: 20.10.2023
(51) Int. Cl.: A61B 1/00, A61B 1/04, G02B 23/24

(54) **ENDOSCOPIC DEVICE**

(30) Priority: 10.11.2022 JP 2022180061
(71) Applicant: HOYA CORPORATION, Shinjuku-ku Tokyo 160-8347 (JP)
(72) Inventor: KOMATSU Masahiro, Tokyo 160-8347 (JP)
(74) Representative: Lucke, Andreas
(86) International application number: PCT/JP2023/037990
(87) International publication number: WO 2024/101114

(57) **Abstract**

Provided is an endoscope device capable of coping with an increase in power without upsizing a connector portion.

The endoscope device includes a processor unit including a power transmitting coil and a control board, and a scope unit including a power receiving coil and an endoscope. While the processor unit and the scope unit are in a joined state to each other, the power transmitting coil and the power receiving coil at least partially overlap each other in an axial direction, and contactless power supply from the power transmitting coil to the power receiving coil is enabled.

## Description

### Technical Field

The present invention relates to an endoscope device.

### Background Art

Some endoscope devices are separated into a processor unit and a scope unit. The scope unit includes an endoscope, and in the endoscope, an imaging module including a large number of constituent parts such as an image sensor and a lens is arranged at a distal tip. A video signal cable, a power supply cable, a control cable, and the like are wired in a flexible tube portion of the scope unit.

A middle-sized electric connector or the like is used to connect the processor unit and the scope unit. A configuration for performing contactless power supply at a connecting portion is known, where a connector of the scope unit includes a power receiving coil and a connector of the processor unit includes a power transmitting coil.

The power consumption of the scope unit may be likely to increase in the future due to enhancement in the resolution of the image sensor, or the like.

Patent Literature 1 describes an endoscope device that performs such contactless power supply.

### Citation List

### Patent Literature

Patent Literature 1: JP 5978238 B2

### Summary of Invention

### Technical Problem

However, the conventional technique has a problem that it is difficult to cope with an increase in power without upsizing the connector portion.

For example, in the configuration of Patent Literature 1, the power transmitting coil and the power receiving coil are arranged so as to abut with their axial end surfaces opposite to each other. In such a configuration, there is a limit to the strength of coupling between the power transmitting coil and the power receiving coil. Therefore, when the power consumption increases, the power may be likely to become insufficient, and it will be necessary to upsize the connector portion.

In addition, also in a case of using a spiral coil (having a swirl shape arranged in a plane), similarly, the power may be likely to become insufficient when the power consumption increases, and it will be necessary to upsize the connector portion.

The present invention has been made to solve such a problem, and an object of the present invention is to provide an endoscope device capable of coping with an increase in power without upsizing a connector portion.

### Solution to Problem

An example of an endoscope device according to the present invention includes:
a processor unit including a power transmitting coil and a control board; and
a scope unit including a power receiving coil and an endoscope;
in which
while the processor unit and the scope unit are in a joined state to each other, the power transmitting coil and the power receiving coil at least partially overlap each other in an axial direction, and contactless power supply from the power transmitting coil to the power receiving coil is enabled.

The present description is based on and claims priority pursuant to Japanese Patent Application No. 2022-180061, the entire disclosure of which is hereby incorporated by reference herein.

### Advantageous Effects of Invention

An endoscope device according to the present invention can cope with an increase in power without upsizing a connector portion.

### Brief Description of Drawings

Fig. 1 is a block diagram of an endoscope device according to a first embodiment of the present invention.
Fig. 2 depicts a more detailed configuration of a processor unit 20 and a scope unit 30.
Fig. 3 is a perspective view illustrating a specific structure of a connector unit 20a included in the processor unit 20.
Fig. 4 is a side view of the connector unit 20a in Fig. 3.
Fig. 5 is a front view of the connector unit 20a in Fig. 3.
Fig. 6 is a perspective view illustrating a specific structure of a connector unit 30a included in the scope unit 30.
Fig. 7 is a side view of the connector unit 30a in Fig. 6.
Fig. 8 is a front view of the connector unit 30a in Fig. 6.
Fig. 9 is a diagram explaining a relationship between a power transmitting coil 21 and a power receiving coil 31.
Fig. 10 is a perspective view illustrating a state of the processor unit 20 and the scope unit 30 joined to each other.
Fig. 11 is a top view illustrating the state of Fig. 10.
Fig. 12 is a side view illustrating the state of Fig. 10.

### Description of Embodiments

Hereinafter, embodiments of the present invention will be described with reference to the accompanying drawings.

### First Embodiment

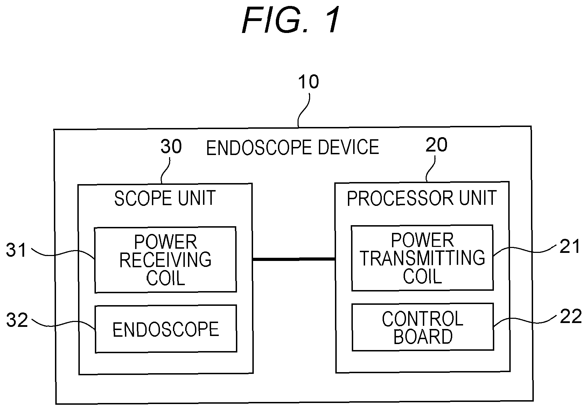

Fig. 1 is a block diagram of an endoscope device according to a first embodiment of the present invention. The endoscope device 10 may be what is called an electronic endoscope, or may be a device specialized for medical use. The endoscope device 10 includes a processor unit 20 and a scope unit 30.

The processor unit 20 includes a power transmitting coil 21 and a control board 22. The control board 22 functions as a control device that controls the processor unit 20 or the entire endoscope device 10 and can be configured using, for example, a computer including a calculation means and a storage means.

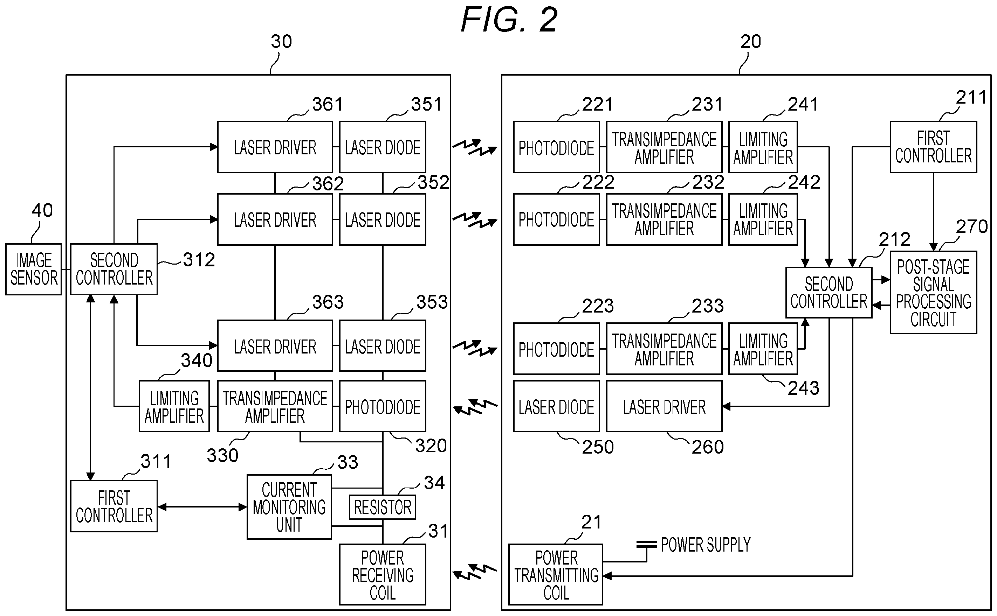

The scope unit 30 includes a power receiving coil 31 and an endoscope 32. The endoscope 32 can have a known configuration and includes, for example, a hard portion that is not deformed, a bent portion that can be actively bent according to an operation, a flexible portion that can be passively deformed, and the like in order from a distal tip. An image sensor (to be described later with reference to Fig. 2 and the like) is attached to the hard portion.

The processor unit 20 or the scope unit 30 may include an operation unit for operating the endoscope 32. A user of the endoscope device 10 can control a movement of the endoscope 32 by operating the operation unit. For example, the bent portion of the endoscope 32 is bent according to an operation on the operation unit. The bent portion can be implemented using a well-known mechanism incorporated in a typical electronic scope, which is configured such that the bent portion is bent by, for example, pulling an operation wire in conjunction with a rotational operation on a knob included in the operation unit.

A portion of the endoscope 32 including the distal tip can be inserted into any body cavity in a living body and, for example, can be inserted into a bronchus, a biliary tract, a pancreas, a hepatic duct region, a urinary organ region, and the like.

The processor unit 20 and the scope unit 30 are attachable to and detachable from each other, which is a configuration that allows the processor unit 20 and the scope unit 30 to be separably joined. While the processor unit 20 and the scope unit 30 are in a joined state to each other, contactless power supply from the power transmitting coil to the power receiving coil is enabled.

Fig. 2 illustrates a more detailed configuration of the processor unit 20 and a scope unit 30. In the processor unit 20, a second controller 212 controls the power transmitting coil 21, thereby controlling the power supplied to the scope unit 30. The power source of the power to be supplied may be a battery built in the processor unit 20 or an external power source.

In addition, the second controller 212 processes signals transmitted to and received from the scope unit 30. A post-stage signal processing circuit 270 processes input and output signals for the second controller 212. A first controller 211 controls the second controller 212 and the post-stage signal processing circuit 270.

In particular, the second controller 212 controls a laser driver 260, and the laser driver 260 controls a laser diode 250. The laser diode 250 transmits an optical signal for use in control to the scope unit 30, thereby controlling a movement of the scope unit 30.

The photodiodes 221, 222, and 223 receive optical signals representing a video (for example, a video captured by an image sensor 40) from the scope unit 30, convert the received optical signals into electric signals, and transmit the converted electric signals to corresponding transimpedance amplifiers 231, 232, and 233 and further to limiting amplifiers 241, 242, and 243 in a subsequent stage, respectively. The limiting amplifiers 241, 242, and 243 amplify the electric signals to transmit the amplified electric signals to the second controller 212. Consequently, the second controller 212 acquires a video signal.

In the scope unit 30, a first controller 311 controls a current monitoring unit 33. The current monitoring unit 33 controls a circuit including the power receiving coil 31 and a resistor 34, thereby supplying power supplied from the processor unit 20 to other portions of the scope unit 30 while controlling the power.

In addition, the first controller 311 receives a video signal from the image sensor 40 and transmits the received video signal to laser drivers 361, 362, and 363. The laser drivers 361, 362, and 363 control corresponding laser diodes 351, 352 and 353, respectively. The laser diodes 351, 352, and 353 transmit optical signals for video signals to the processor unit 20. The optical signals from the laser diodes 351, 352, and 353 of the scope unit 30 are received by, for example, the photodiodes 221, 222, and 223 of the processor unit 20, respectively.

The photodiode 320 receives the optical signal for use in control from the processor unit 20, converts the received optical signal into an electric signal, and transmits the converted electric signal to a transimpedance amplifier 330 and further to a limiting amplifier 340 in a subsequent stage. The limiting amplifier 340 amplifies the electric signal and transmits the amplified electric signal to a second controller 312. Consequently, the second controller 312 receives a control signal.

In the example in Fig. 2, the image sensor 40 is not included in the scope unit 30, but as a modification, the image sensor 40 may be included in the scope unit 30. Note that, although not illustrated in Fig. 2, each of the processor unit 20 and the scope unit 30 includes an optical path for illumination light (to be described later). Light for illuminating an object to be imaged by the image sensor 40 is conveyed via these optical paths.

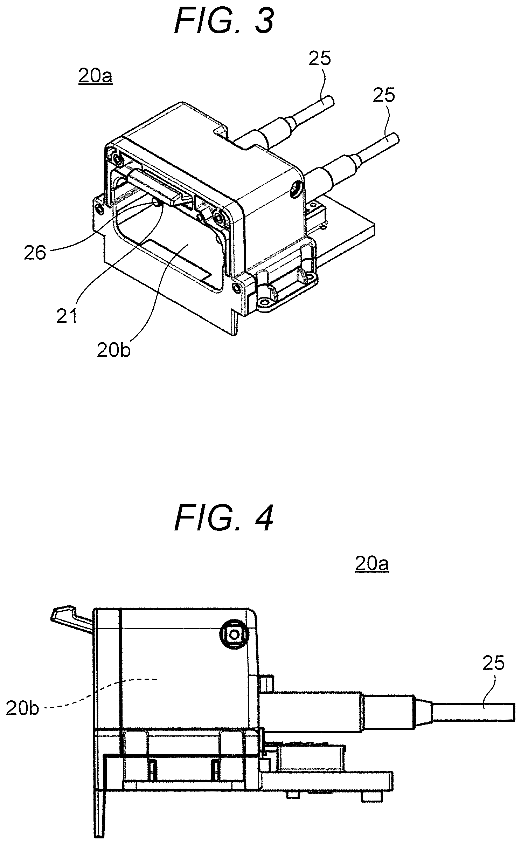

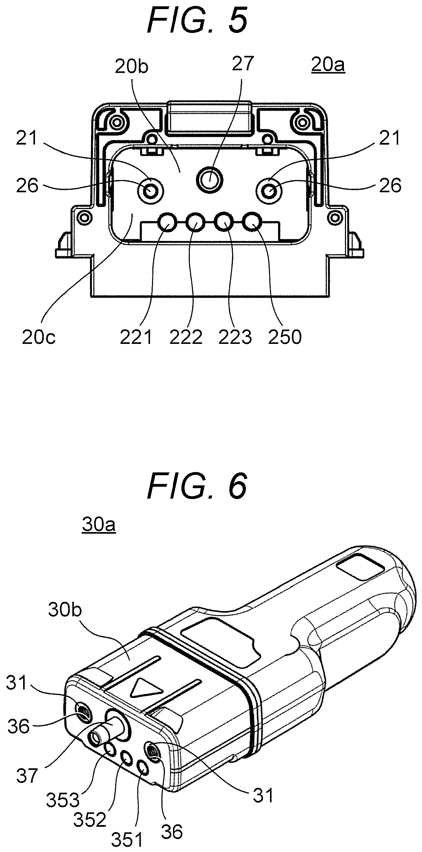

Figs. 3 to 5 illustrate a specific structure of a connector unit 20a included in the processor unit 20. Fig. 3 is a perspective view, Fig. 4 is a side view, and Fig. 5 is a front view.

The connector unit 20a has a fitting recess 20b, which is configured such that a fitting protrusion 30b (to be described later with reference to Figs. 6 to 8) of the scope unit 30 can be fitted into the fitting recess 20b. A coil support protrusion 26 is formed in the fitting recess 20b. The coil support protrusion 26 has a cylindrical protruding surface extending in a predetermined axial direction, and the power transmitting coil 21 is arranged along the protruding surface (only the position of the power transmitting coil 21 is illustrated in Figs. 3 and 5). That is, the power transmitting coil 21 is wound around the coil support protrusion 26.

The power transmitting coil 21 is formed not in a spiral shape but in a solenoid shape. Note that, in the present description, the spiral shape signifies, for example, a swirl shape in which a coil is wound while a winding diameter is changed in a plane, and the solenoid shape signifies, for example, a helical shape in which a coil is wound while proceeding in an axial direction in a cylindrical surface. As described above, in the present embodiment, the power transmitting coil 21 is wound along a cylindrical surface.

A power transmission cable 25 is connected to the power transmitting coil 21, and power is supplied to the power transmitting coil 21 via the power transmission cable 25. In the present embodiment, the processor unit 20 includes two power transmitting coils 21.

In addition, the photodiodes 221, 222, and 223 and the laser diode 250 described above are arranged to face the fitting recess 20b (Fig. 5). Note that the processor unit 20 may further include an optical transmission member that transmits light between those components and the outside, and such an optical transmission member may be arranged to face the fitting recess 20b.

The processor unit 20 includes an optical path 27 for illumination light, and the optical path 27 is arranged to face the fitting recess 20b. The optical path 27 guides illumination light from a light source arranged inside or outside the processor unit 20 to the fitting recess 20b.

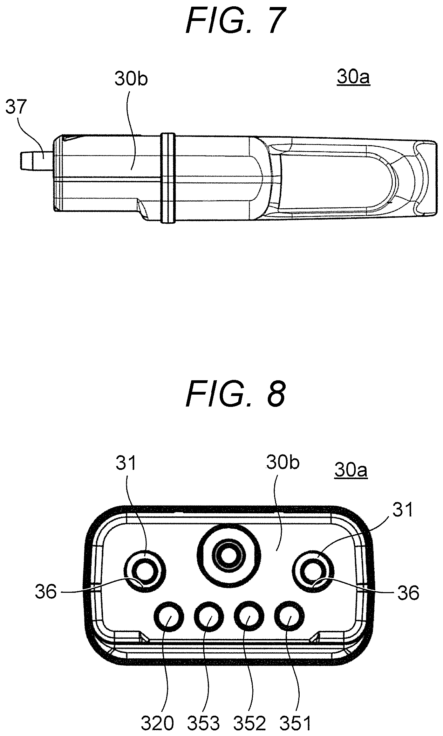

Figs. 6 to 8 illustrate a specific structure of a connector unit 30a included in the scope unit 30. Fig. 6 is a perspective view, Fig. 7 is a side view, and Fig. 8 is a front view.

The connector unit 30a has the fitting protrusion 30b. As described above, the fitting protrusion 30b can be fitted into the fitting recess 20b of the processor unit 20. A coil support recess 36 is formed in the fitting protrusion 30b. The coil support recess 36 has a cylindrical recessed surface extending in a predetermined axial direction, and the power receiving coil 31 is arranged along the recessed surface (only the position of the power receiving coil 31 is illustrated in Figs. 6 and 8). That is, the power receiving coil 31 is wound around the coil support recess 36.

Similarly to the power transmitting coil 21, the power receiving coil 31 is also formed in a solenoid shape instead of a spiral shape. That is, in the present embodiment, the power receiving coil 31 is also wound along a cylindrical surface.

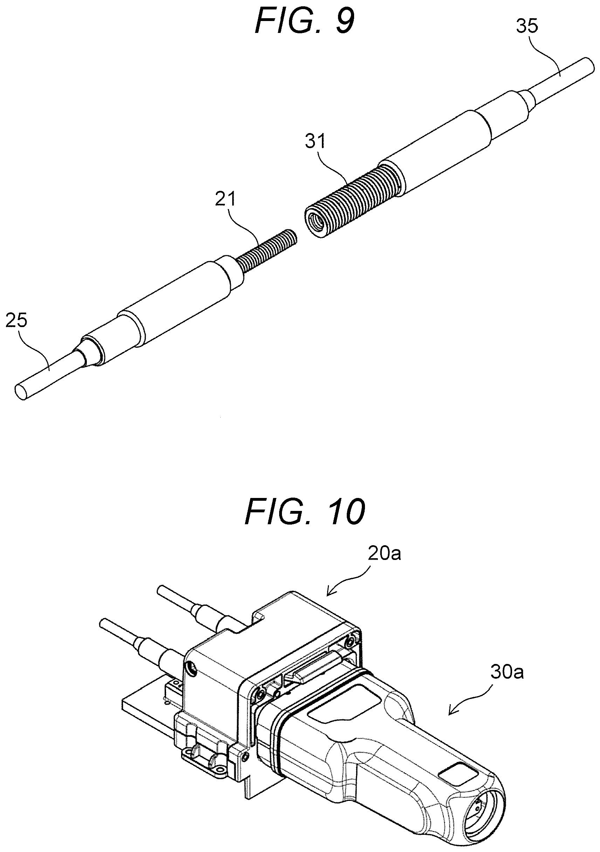

A power transmission cable 35 (not illustrated in Figs. 6 to 8, see Fig. 9) is connected to the power receiving coil 31, and power is supplied from the power receiving coil 31 to other portions of the scope unit 30 via the power transmission cable 35. In the present embodiment, the scope unit 30 includes two power receiving coils 31.

In addition, the photodiode 320 and the laser diodes 351, 352, and 353 are arranged on an end surface of the fitting protrusion 30b. Note that the scope unit 30 may further include an optical transmission member that transmits light between these components and the outside, and such an optical transmission member may be arranged on an end surface of the fitting protrusion 30b.

The scope unit 30 includes an optical path 37 for illumination light, and the optical path 37 is arranged via an end surface of the fitting protrusion 30b. The optical path 37 conveys illumination light conveyed from the processor unit 20 to other portions of the scope unit 30 (for example, an illumination unit arranged at a distal tip or in the vicinity of the image sensor 40).

A relationship between the power transmitting coil 21 and the power receiving coil 31 will be described with reference to Fig. 9. An outer diameter of the power transmitting coil 21 is formed to be smaller than an inner diameter of the power receiving coil 31, and the power transmitting coil 21 can be inserted into the power receiving coil 31 so as to overlap each other (partially or entirely) in an axial direction. In the present embodiment, by fitting the fitting protrusion 30b of the scope unit 30 into the fitting recess 20b of the processor unit 20, the coil support protrusion 26 of the processor unit 20 is inserted into the coil support recess 36 of the scope unit 30, whereby the power transmitting coil 21 is inserted into the power receiving coil 31.

In this manner, while the processor unit 20 and the scope unit 30 are in a joined state to each other, the power transmitting coil 21 and the power receiving coil 31 at least partially overlap each other in the axial direction, and contactless power supply from the power transmitting coil 21 to the power receiving coil 31 is enabled. The approach of the contactless power supply can be, for example, an electromagnetic induction approach, but may use a resonance approach or another approach.

As described above, the power transmitting coil 21 and the power receiving coil 31 having the solenoid shape are used and configured such that the power supply is performed while the power transmitting coil 21 and the power receiving coil 31 are in an overlapping state with each other at least partially in the axial direction, whereby relatively large power can be supplied. Therefore, the endoscope device 10 according to the first embodiment of the present invention can cope with an increase in power without upsizing the connector portion.

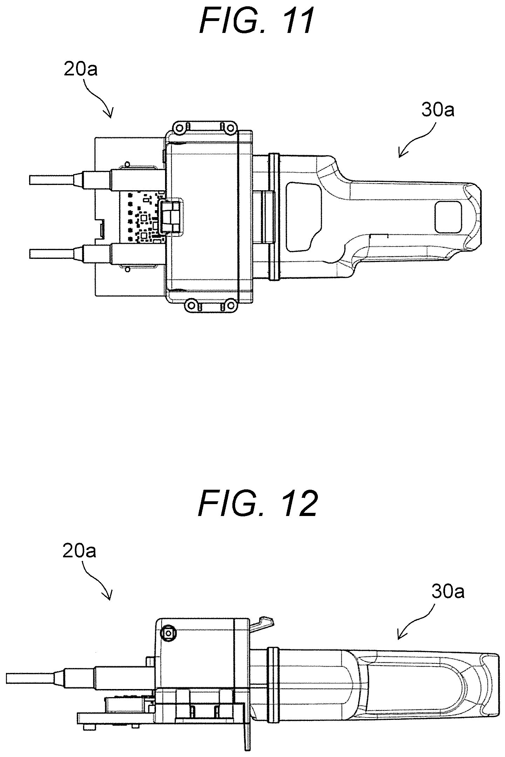

Figs. 10 to 12 illustrate a state of the processor unit 20 and the scope unit 30 (more specifically, the connector unit 20a of the processor unit 20 and the connector unit 30a of the scope unit 30) joined to each other. Fig. 10 is a perspective view, Fig. 11 is a top view, and Fig. 12 is a side view.

As described above, in a state of the processor unit 20 and the scope unit 30 joined to each other, the cylindrical protruding surface of the coil support protrusion 26 and the cylindrical recessed surface of the coil support recess 36 are fitted to each other. Furthermore, in this state, the power transmitting coil 21 and the power receiving coil 31 overlap each other in the axial direction, and contactless power supply from the power transmitting coil 21 to the power receiving coil 31 is enabled, as described above.

In addition, in this state, transmission and reception of optical signals are enabled by the laser diode 250 of the processor unit 20 and the photodiode 320 of the scope unit 30, whereby the control signal for the scope unit 30 is transmitted. Furthermore, in this state, transmission and reception of optical signals are enabled by the laser diodes 351, 352, and 353 of the scope unit 30 and the photodiodes 221, 222, and 223 of the processor unit 20, whereby the video signal is transmitted to the processor unit 20.

Moreover, in this state, the optical path 27 for illumination light of the processor unit 20 and the optical path 37 for illumination light of the scope unit 30 are joined. Consequently, the illumination light is transmitted to the scope unit 30.

### Modifications

Various modifications can be made in the above-described first embodiment within the scope of the present invention. As a specific example, the following modifications are possible.

In the first embodiment, the number of each of the power transmitting coils 21 and the power receiving coils 31 is two, but may be one, or may be three or more.

In the first embodiment, the processor unit 20 and the scope unit 30 are joined by the fitting recess 20b and the fitting protrusion 30b fitting to each other, but also can be joined by an approach other than fitting.

In addition, in a case of joint by fitting, the recess and the protrusion can also be replaced with each other. For example, the processor unit 20 may have a fitting protrusion and a coil support recess, and the scope unit 30 may have a fitting recess and a coil support protrusion.

Transmission and reception of the control signal and/or the video signal between the processor unit 20 and the scope unit 30 are not limited to transmission and reception using the optical signal. In addition, in a case where the optical signal is used, a transfer direction of the optical signal may be from the processor unit 20 toward the scope unit 30, or conversely, may be from the scope unit 30 toward the processor unit 20 (in the first embodiment, both of the directions are included).

The processor unit 20 may include a joint detection switch that detects that the processor unit 20 and the scope unit 30 have been joined to each other. Instead of or in addition to the processor unit 20, the scope unit 30 may include such a joint detection switch.

As a specific example of the joint detection switch, a switch plate 20c illustrated in Fig. 5 can be used. The switch plate 20c is arranged on an end surface of the fitting recess 20b of the processor unit 20 and is biased toward the inside of the fitting recess 20b by, for example, a spring. When the processor unit 20 and the scope unit 30 are joined to each other, the switch plate 20c is pushed and moved by the fitting protrusion 30b of the scope unit 30, whereby the joint can be detected. Note that the specific structure of the joint detection switch is not limited to the plate-like one as described above.

The processor unit 20 and the scope unit 30 may include a metal connection path for discharging static electricity. This path may be provided separately from the joint detection switch and a signal ground.

The present disclosure includes the following specifying matters.

### [Specifying Matter 1]

An endoscope device including:
a processor unit including a power transmitting coil and a control board; and
a scope unit including a power receiving coil and an endoscope;
in which
while the processor unit and the scope unit are in a joined state to each other, the power transmitting coil and the power receiving coil at least partially overlap each other in an axial direction, and contactless power supply from the power transmitting coil to the power receiving coil is enabled.

### [Specifying Matter 2]

The endoscope device according to the specifying matter 1, in which the power transmitting coil and the power receiving coil are wound along a cylindrical surface.

### [Specifying Matter 3]

The endoscope device according to the specifying matter 1, in which
one of the processor unit and the scope unit includes a protruding surface having a cylindrical shape,
the other of the processor unit and the scope unit includes a recessed surface having a cylindrical shape,
the power transmitting coil and the power receiving coil are both arranged along the protruding surface or the recessed surface, and
the protruding surface and the recessed surface are fitted to each other while the processor unit and the scope unit are in a joined state to each other.

### [Specifying Matter 4]

The endoscope device according to the specifying matter 1, in which
the processor unit includes a plurality of the power transmitting coils, and
the scope unit includes a plurality of the power receiving coils.

### [Specifying Matter 5]

The endoscope device according to the specifying matter 1, in which
one of the processor unit and the scope unit includes a light-emitting element,
the other of the processor unit and the scope unit includes a light-receiving element, and
the light-emitting element and the light-receiving element are allowed to transmit and receive an optical signal while the processor unit and the scope unit are in a joined state to each other.

### [Specifying Matter 6]

The endoscope device according to the specifying matter 1, in which
each of the processor unit and the scope unit includes an optical path for illumination light, and
the optical paths of the processor unit and the scope unit are joined while the processor unit and the scope unit are in a joined state to each other.

### [Specifying Matter 7]

The endoscope device according to the specifying matter 1, in which at least one of the processor unit and the scope unit includes a joint detection switch that detects that the processor unit and the scope unit have been joined to each other.

### Reference Signs List

- 10: Endoscope device
- 20: Processor unit
- 21: Power transmitting coil
- 22: Control board
- 25: Power transmission cable
- 26: Coil support protrusion
- 27: Optical path for illumination light
- 30: Scope unit
- 31: Power receiving coil
- 32: Endoscope
- 33: Current monitoring unit
- 34: Resistor
- 35: Power transmission cable
- 36: Coil support recess
- 37: Optical path for illumination light
- 40: Image sensor
- 20a: Connector unit
- 20b: Fitting recess
- 20c: Switch plate
- 211: First controller
- 212: Second controller
- 221 to 223: Photodiode (light-receiving element)
- 231 to 233: Transimpedance amplifier
- 241 to 243: Limiting amplifier
- 250: Laser diode (light-emitting element)
- 260: Laser driver
- 270: Post-stage signal processing circuit
- 30a: Connector unit
- 30b: Fitting protrusion
- 311: First controller
- 312: Second controller
- 320: Photodiode (light-receiving element)
- 330: Transimpedance amplifier
- 340: Limiting amplifier
- 351 to 353: Laser diode (light-emitting element)
- 361 to 363: Laser driver

All publications, patents, and patent applications cited herein are hereby incorporated by reference in their entirety.

## Claims

1. An endoscope device comprising:
a processor unit including a power transmitting coil and a control board; and
a scope unit including a power receiving coil and an endoscope;
wherein
while the processor unit and the scope unit are in a joined state to each other, the power transmitting coil and the power receiving coil at least partially overlap each other in an axial direction, and contactless power supply from the power transmitting coil to the power receiving coil is enabled.

2. The endoscope device according to claim 1, wherein the power transmitting coil and the power receiving coil are wound along a cylindrical surface.

3. The endoscope device according to claim 1, wherein
one of the processor unit and the scope unit includes a protruding surface having a cylindrical shape,
the other of the processor unit and the scope unit includes a recessed surface having a cylindrical shape,
the power transmitting coil and the power receiving coil are both arranged along the protruding surface or the recessed surface, and
the protruding surface and the recessed surface are fitted to each other while the processor unit and the scope unit are in a joined state to each other.

4. The endoscope device according to claim 1, wherein
the processor unit includes a plurality of the power transmitting coils, and
the scope unit includes a plurality of the power receiving coils.

5. The endoscope device according to claim 1, wherein
one of the processor unit and the scope unit includes a light-emitting element,
the other of the processor unit and the scope unit includes a light-receiving element, and the light-emitting element and the light-receiving element are allowed to transmit and receive an optical signal while the processor unit and the scope unit are in a joined state to each other.

6. The endoscope device according to claim 1, wherein
each of the processor unit and the scope unit includes an optical path for illumination light, and
the optical paths of the processor unit and the scope unit are joined while the processor unit and the scope unit are in a joined state to each other.

7. The endoscope device according to claim 1, wherein at least one of the processor unit and the scope unit includes a joint detection switch that detects that the processor unit and the scope unit have been joined to each other.
